# EUROPEAN PATENT APPLICATION

(11) **EP 3 943 018 A1**
(43) Date of publication of application: **26.01.2022**
(21) Application number: 21186438.4
(22) Date of filing: 19.07.2021
(51) Int. Cl.: A61B 17/072

(54) **SURGICAL STAPLING DEVICE WITH TISSUE GAP CONTROL AND CONTROLLED STAPLE FORMATION**

(30) Priority: 20.07.2020 US 202016933461
(71) Applicant: Covidien LP, Mansfield, MA 02048 (US)
(72) Inventor: BEARDSLEY, John W., Wallingford, Connecticut 06492 (US); MARCZYK, Stanislaw Z., Stratford, Connecticut 06614 (US)
(74) Representative: Maschio, Antonio

(57) **Abstract**

A surgical stapling device includes tool assembly having an anvil and a cartridge assembly. The cartridge assembly includes a channel member that defines a cavity, a staple cartridge supported within the cavity of the channel member, and a biasing member. The staple cartridge supports staples, each having a back span and a pair of legs depending from the back span. The biasing member is positioned within the channel member and is deformable to allow the cartridge body to move vertically within the cavity of the channel member. The anvil incudes staple deforming pockets that are configured to deform the legs of the staples from a position within the same plane as the back span to positions laterally offset from the back span.

## Description

### FIELD

This disclosure is directed to surgical stapling devices and, more particularly, to surgical stapling devices that have a tool assembly with tissue gap control and controlled staple formation.

### BACKGROUND

Surgical stapling devices for ejecting staples to join tissue or tissue segments in a fast and efficient manner in a variety of surgical procedures, e.g., anastomoses procedures, are well known. Typically, a surgical stapling device includes a tool assembly having first and second jaws that support a cartridge assembly and an anvil, respectively. The cartridge assembly includes a staple cartridge that supports a plurality of staples. The first and second jaws are mounted together to allow movement of the tool assembly between open and clamped positions. In the clamped position, the staple cartridge of the cartridge assembly and the anvil are supported in juxtaposed alignment and include tissue contact surfaces that define a predetermined tissue gap. The tissue gap and the size of the staples within the staple cartridge are dimensioned to receive and suture tissue having a thickness within a predetermined range to properly effect hemostasis. If the tissue positioned between the jaws is outside the predetermined range, i.e., either too thick or too thin, the stapling device may not provide effective hemostasis. In addition, if the tissue thickness is misidentified by the clinician or if the tissue thickness falls near the outer edges of the range for a given staple size, the likelihood of ineffective hemostasis is increased.

Accordingly, a continuing need exists in the suturing arts for a surgical stapling device that can provide effective hemostasis for a greater range of tissue thicknesses.

### SUMMARY

One aspect of the disclosure is directed to an anvil for a surgical stapling device that includes a body defining a longitudinal axis and having a tissue engaging surface. The tissue engaging surface defines a central knife slot and a series of staple forming pockets positioned on each side of the central knife slot. Each of the staple forming pockets includes first and second concavities. The first and second concavities are positioned to receive first and second legs, respectively, of a staple including a back span. The first and second concavities are configured to form the legs of a respective staple into a B-shape with the legs laterally offset from the back span.

Another aspect of the disclosure is directed to a tool assembly including a cartridge assembly and an anvil. The cartridge assembly includes a staple cartridge and a channel member. The channel member has a bottom wall and side walls defining a cavity. The staple cartridge includes a cartridge body that defines a longitudinal axis, pushers, staples, and an actuation sled. The cartridge body defines a central knife slot and a series of staple receiving pockets on each side of the central knife slot. Each of the staples includes a back span and first and second legs secured to the back span. Each of the staples is received within one of the series of staple receiving slots. The first and second legs of each of the staples and the back span of the respective staples are aligned in a common plane. The anvil includes a body that defines a longitudinal axis and has a tissue engaging surface. The tissue engaging surface defines a central knife slot and a series of staple forming pockets positioned on each side of the central knife slot. Each of the staple forming pockets includes first and second concavities. The first and second concavities are positioned to receive the first and second legs, respectively, of a respective one of the staples and are configured to form the legs into a B-shape with the first and second legs of the staples laterally offset from the back span.

In aspects of the disclosure, each of the first and second concavities includes tapered side walls and a linear channel, and the tapered side walls are configured to direct the legs of the staple into the linear channel.

In some aspects of the disclosure, the linear channel of each of the first concavities defines an angle Ω1 with the longitudinal axis of the body of the anvil, and the linear channel of each of the second concavities defines an angle Ω2 with the longitudinal axis of the body of the anvil.

In certain aspects of the disclosure, the angles Ω1 and Ω2 are from about 5 degrees to about 30 degrees.

In aspects of the disclosure, each of the first and second concavities is tear-drop shaped.

In some aspects of the disclosure, each of the first and second concavities has a wall that is angled downwardly in a direction orthogonal to the longitudinal axis of the body of the anvil.

In certain aspects of the disclosure, each of the first and second concavities is linear.

In aspects of the disclosure, the cartridge assembly includes a biasing member that is positioned on the bottom wall of the channel member between the cartridge body and the channel member, and the biasing member deformable to allow for vertical movement of the cartridge body within the cavity.

In some aspects of the disclosure, the cartridge body includes tabs and the channel member defines windows that receive the tabs, and the tabs are movable vertically within the windows to allow the cartridge body to move vertically in relation to the channel member.

In certain aspects of the disclosure, the biasing member is selected from the group consisting of compression springs and leaf springs.

Another aspect of the disclosure is directed to a surgical stapling device including a handle assembly, an adapter assembly, and a tool assembly. The adapter assembly has a proximal portion and a distal portion. The proximal portion is coupled to the handle assembly the tool assembly is supported on the distal portion of the adapter assembly. The tool assembly includes a cartridge assembly and an anvil. The cartridge assembly includes a staple cartridge and a channel member. The channel member has a bottom wall and side walls that define a cavity. The staple cartridge includes a cartridge body defining a longitudinal axis, pushers, staples, and an actuation sled. The cartridge body defines a central knife slot and a series of staple receiving pockets on each side of the central knife slot. Each of the staples includes a back span and first and second legs that are secured to the back span. Each of the staples is received within one of the series of staple receiving slots. The first and second legs of each of the staples and the back span of the respective staple are aligned in a common plane within the cartridge body. The anvil includes a body that defines a longitudinal axis and has a tissue engaging surface. The tissue engaging surface defines a central knife slot and a series of staple forming pockets that are positioned on each side of the central knife slot. Each of the staple forming pockets includes first and second concavities that are positioned to receive the first and second legs, respectively, of a respective one of the staples and are configured to form the legs into a B-shape with the first and second legs of the staples laterally offset from the back span.

Other aspects of the disclosure will be appreciated from the following description.

### BRIEF DESCRIPTION OF THE DRAWINGS

Various aspects of the disclosed stapling device are described herein below with reference to the drawings, wherein:
FIG. 1 is a side perspective view of a surgical stapling device including aspects of the disclosure with a tool assembly of the stapling device in an open position;
FIG. 2 is an enlarged view of the indicated area of detail shown in FIG. 1;
FIG. 3 is a side perspective view of a cartridge assembly of the tool assembly shown in FIG. 2;
FIG. 4 is a side perspective view of the cartridge assembly shown in FIG. 3 with a staple cartridge separated from a channel member of the cartridge assembly;
FIG. 5 is an exploded view of the cartridge assembly shown in FIG. 4;
FIG. 5A is a side perspective view of an alternative version of a biasing member of the cartridge assembly shown in FIG. 5;
FIG. 6 is a side perspective view of an anvil of the tool assembly shown in FIG. 1;
FIG. 7 is an enlarged view of the indicated area of detail shown in FIG. 6 showing a staple forming surface of the anvil;
FIG. 8 is a cutaway plan view of the staple forming surface of the anvil shown in FIG. 6;
FIG. 9A is a side perspective view of a staple;
FIG. 9B is a side perspective view of the staple shown in FIG. 9A formed by the anvil shown in FIG. 6;
FIG. 10 is a bottom view of the formed staple shown in FIG. 9B;
FIG. 11 is a cutaway plan view of an alternative version of the staple forming surface of the anvil shown in FIG. 6;
FIG. 12 is a side perspective view of a staple formed by the anvil shown in FIG. 11;
FIG. 13 is a cutaway perspective view of another alternative version of the staple forming surface of the anvil shown in FIG. 6;
FIG. 14 is a cutaway plan view of yet another alternative version of the staple forming surface of the anvil shown in FIG. 6;
FIG. 15 is a cross-sectional view taken along section line 15-15 of FIG. 14;
FIG. 16 is a cutaway perspective view from a first side of another alternative version of the staple forming surface of the anvil shown in FIG. 6;
FIG. 17 is a is a cutaway perspective view of another alternative version of the staple forming surface of the anvil shown in FIG. 6;
FIG. 18 is a cross-sectional view taken along section line 18-18 of FIG. 17; and
FIG. 19 is a cross-sectional view taken along section line 19-19 of FIG. 18.

### DETAILED DESCRIPTION

The disclosed surgical stapling device will now be described in detail with reference to the drawings in which like reference numerals designate identical or corresponding elements in each of the several views. However, it is to be understood that the disclosed aspects are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through a small diameter incision or cannula. Further, the term "clinician" is used generally to refer to medical personnel including doctors, nurses, and support personnel. Moreover, directional terms such as front, rear, upper, lower, top, bottom, and similar terms are used to assist in understanding the description and are not intended to limit the disclosure.

The disclosed surgical stapling device includes a tool assembly having an anvil, and a cartridge assembly that includes a cartridge channel, a staple cartridge supported within the cartridge channel, and a biasing member. The cartridge channel has a pair of spaced sidewalls and a bottom wall. The biasing member is supported within the cartridge channel between the cartridge channel and the staple cartridge and allows the staple cartridge to move or float within the cartridge channel to accommodate tissues of different thicknesses. The disclosed surgical stapling device also includes an anvil that has a staple forming surface to control formation of staples ejected from the staple cartridge. In aspects of the disclosure, the staple forming surface of the anvil includes forming pockets that are configured to deform legs of the staples into a substantially B-shaped configuration with the legs positioned to one side of a back span of the staples.

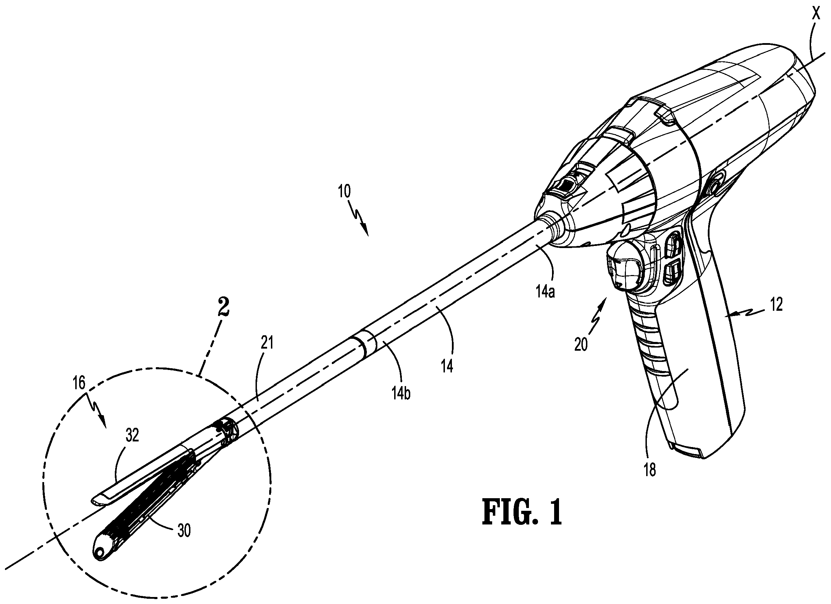

FIG. 1 illustrates a surgical stapling device shown generally as stapling device 10 that includes a handle assembly 12, an elongate body or adapter assembly 14, and a tool assembly 16. As illustrated, the handle assembly 12 is powered and includes a stationary handgrip 18 and actuation buttons 20. The actuation buttons 20 are operable to actuate various functions of the tool assembly 16 via the adapter assembly 14 including approximation, stapling, and cutting. In certain aspects of the disclosure, the handle assembly 12 supports batteries (not shown) that provide power to the handle assembly 12 to operate the stapling device 10. Although the stapling device 10 is illustrated as a powered stapling device, it is envisioned that the disclosed tool assembly 16 is suitable for use with manually powered surgical stapling devices as well as robotically controlled stapling devices.

The adapter assembly 14 includes a proximal portion 14a and a distal portion 14b. The proximal portion 14a is coupled to the handle assembly 12 and the distal portion 14b supports the tool assembly 16. In aspects of the disclosure, the tool assembly 16 forms part of a reload assembly 21 that is removably supported on the distal portion 14b of the adapter assembly 14 and can be replaced after the stapling device 10 is fired to facilitate reuse of the stapling device 10. It is also envisioned that the tool assembly 16 can be fixedly coupled to the distal portion 14b of the adapter assembly 14. The tool assembly 16 of the stapling device 10 includes a cartridge assembly 30 and an anvil 32. The cartridge assembly 30 and the anvil 32 are coupled together such that the tool assembly 16 can pivot between an open position (FIG. 1) and a clamped position. In the clamped position, the anvil 32 and the cartridge assembly 30 are in juxtaposed alignment with each other.

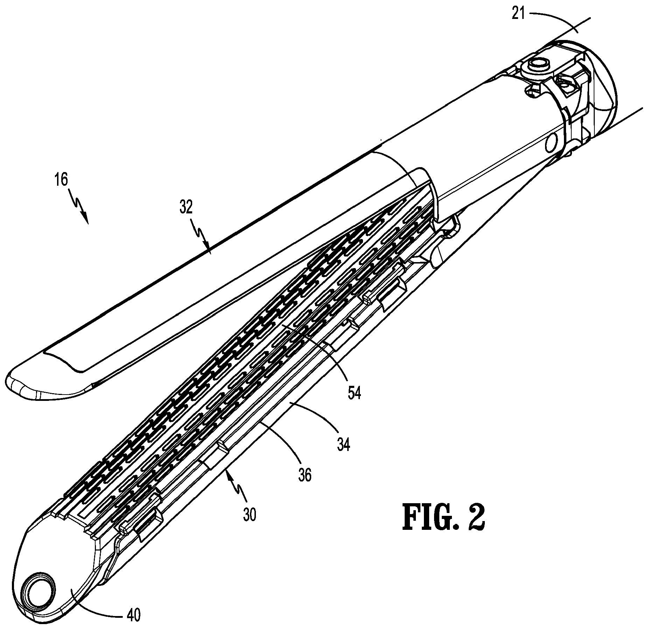

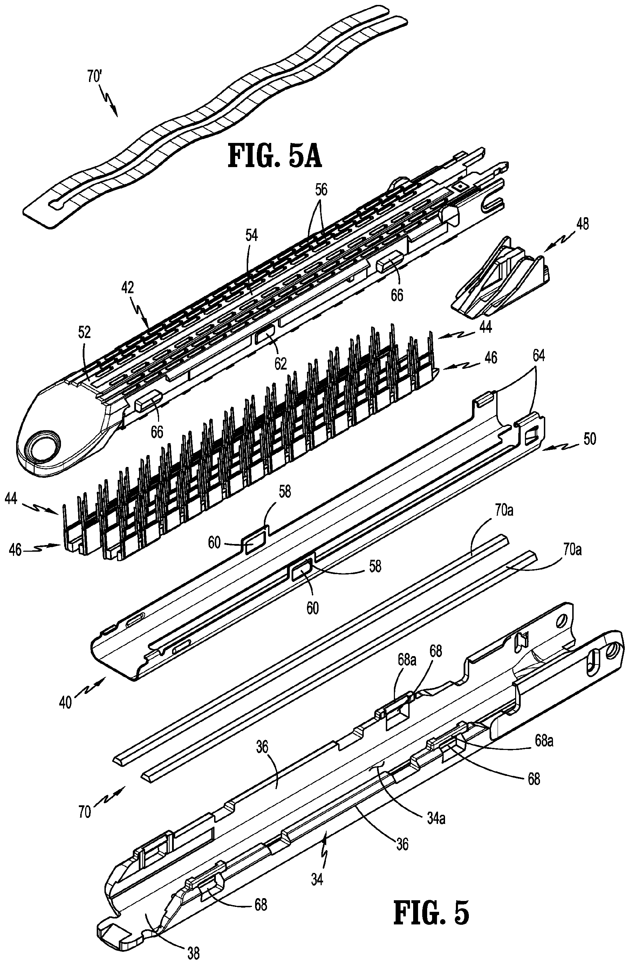

FIGS. 2-5 illustrate the cartridge assembly 30 which includes a channel member 34 and a staple cartridge 40. The channel member 34 includes side walls 36 and a bottom wall 38 that define a cavity 34a (FIG. 5) that receives the staple cartridge 40. In aspects of the disclosure, the staple cartridge 40 can be removably received within the cavity 34a of the channel member 34 to allow for replacement of the staple cartridge 40 after each firing of the stapling device 10 to facilitate reuse of the stapling device 10 (FIG. 1).

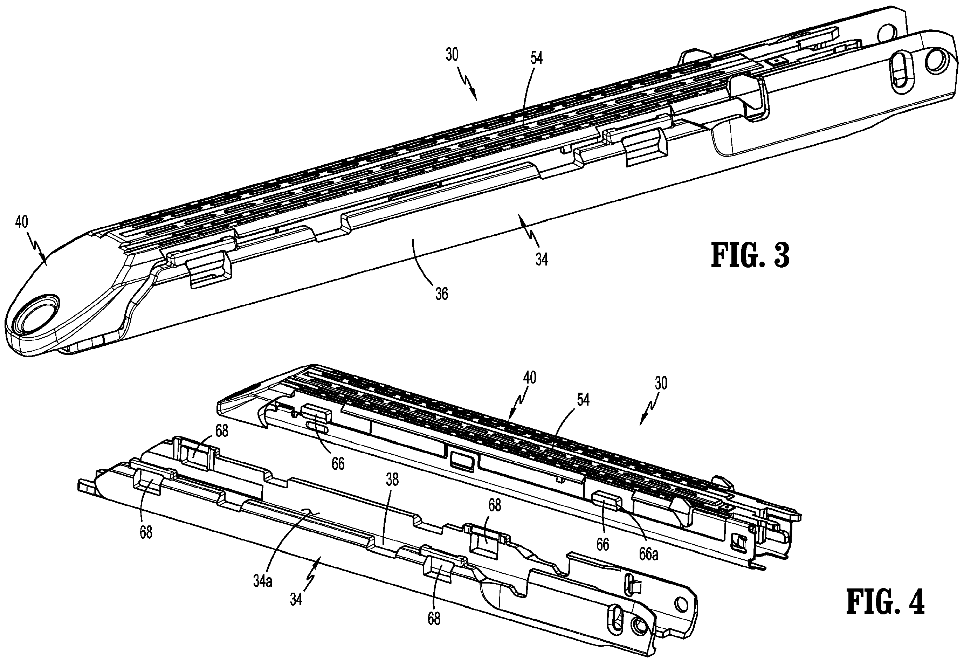

FIG. 5 illustrates an exploded view of the staple cartridge 40 which includes a cartridge body 42, staples 44, pushers 46, an actuation sled 48, and a staple guard 50. The cartridge body 42 of the staple cartridge 40 is received within the cavity 34a defined by the channel member 34 and includes a tissue engaging surface 52. The cartridge body 42 defines a central knife slot 54 and a plurality of staple receiving pockets 56 that are arranged in rows on each side of the central knife slot 54. In certain aspects of the disclosure, the tissue engaging surface 52 of the staple cartridge 40 has a stepped configuration with a raised surface adjacent the central knife slot 54 and lower surfaces positioned outwardly towards the side walls 36 of the channel member 34. Each of the staple receiving pockets 56 receives a staple 44 (FIG. 3) and at least a portion of one of the pushers 46. In some aspects of the disclosure, the staples 44 in the inner rows of staple receiving pockets 56 are shorter in length than the staples 46 in the outer rows of staple receiving pockets 56. The actuation sled 48 is movable from a proximal end of the cartridge body 42 distally into sequential engagement with the pushers 46 to urge the pushers 46 upwardly as viewed in FIG. 5 to eject the staples 44 from the staple receiving pockets 56 of the cartridge body 42.

The staple guard 50 is secured to a bottom side of the cartridge body 42 to retain the staples 44, pushers 46, and the actuation sled 48 within the cartridge body 42. In aspects of the disclosure, the staple guard 50 includes flexible tabs 58 that define openings 60 that receive protrusions 62 formed on an outer surface of the cartridge body 42 to secure the staple guard 50 to the cartridge body 42. In aspects of the disclosure, the staple guard 50 also includes hooked fingers 64 that engage a proximal portion of the cartridge body 42 to secure the staple guard 50 to the cartridge body 42. U.S. Patent No. 6,241,139 discloses exemplary aspects of the construction and operation of a staple cartridge of a surgical stapling device.

The cartridge body 42 of the staple cartridge 40 includes tabs 66 and the channel member defines slots 68 that receive the tabs 66. The slots 68 are dimensioned to allow the cartridge body 42 of the staple cartridge 40 to move vertically within the slots 68 but prevent longitudinal movement of the staple cartridge 40 within the cavity 34a of the channel member 34. In aspects of the disclosure, the slots 68 are enclosed by bars 68a to define windows that prevent the tabs 66 from moving vertically from within the slots 68. In aspects of the disclosure, the tabs 66 can have convex side walls 66a that allow the cartridge body 40 to rock back and forth within the cavity 34a of the channel member 34.

The cartridge assembly 30 includes a biasing member or members 70 that is/are positioned between the staple guard 50 and the bottom wall 38 of the channel member 34. The biasing member(s) urges the staple cartridge 40 upwardly within the cavity 34a of the channel member 34 towards the anvil 32. In aspects of the disclosure, the biasing member 70 can be in the form of compression springs 70a and 70b, e.g., polymer compression springs (FIG. 5), that are positioned in spaced relation to each other on the bottom wall 38 of the channel member 34 between the staple guard 50 of the staple cartridge 40 and the bottom wall 38 of the channel member 34. In an alternative version of the biasing member 70, the biasing member 70 can be in the form of an elongated leaf spring 70' (FIG. 5A) that is positioned on the bottom wall 38 of the channel member 34 between the staple guard 50 of the staple cartridge 40 and the bottom wall 38 of the channel member 34.

When tissue is positioned between the anvil 32 and the cartridge assembly 30 and the tool assembly 16 is moved from the open position to the clamped position, the pressure of the tissue on the cartridge body 42 of the staple cartridge 40 compresses the biasing member 70 , 70' to move the staple cartridge 40 vertically (or towards the bottom wall 38 of the channel member 34) within the cavity 34a of the channel member 34. The amount of movement of the staple cartridge 40 within the cavity 34a of the channel member 34 will depend on the thickness of the tissue. More specifically, the staple cartridge 40 will move downwardly within the cavity 34a of the channel member 34 a greater distance as the tissue thickness increases. As the staple cartridge 40 moves within the channel member 34, the tabs 66 move within the slots 68 defined in the side walls 36 of the channel member 34. As described above, the tabs 66 and the windows 68 allow the entire staple cartridge 40 to move vertically within the cavity 34a defined by the channel member 34 but prevent axial movement of the staple cartridge 0 within the cavity 34a. As the entire staple cartridge 40 moves within the channel member 34, the relative position of the pushers 46 change in relation to the anvil 32 which affects staple formation. More specifically, when thick tissue is clamped, the staple cartridge 40 is compressed to a maximum tissue gap to properly form staples. When thinner tissue is clamped, the staple cartridge 40 (including the pushers 46) is closer to the anvil 32 which allows the legs of the staples to curl as described in further detail below.

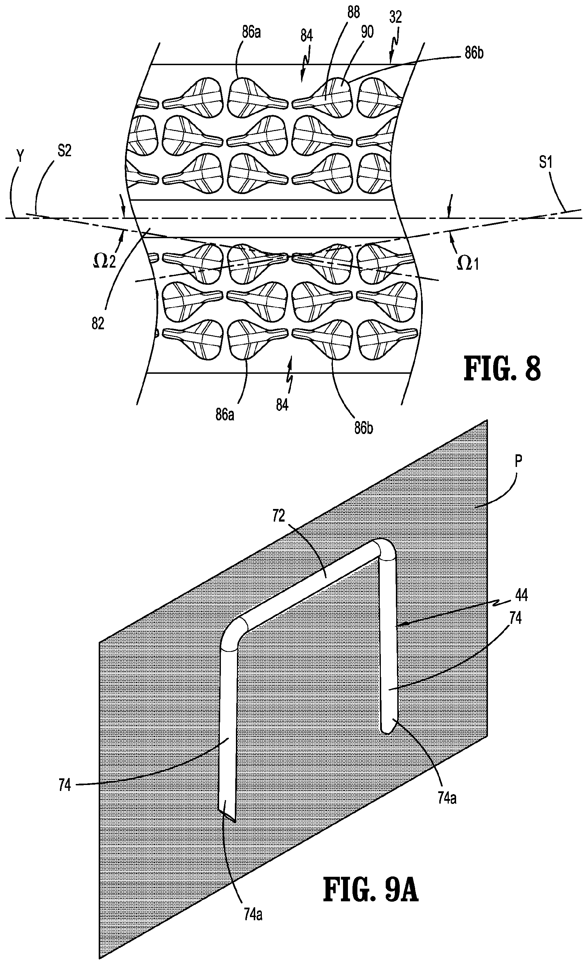

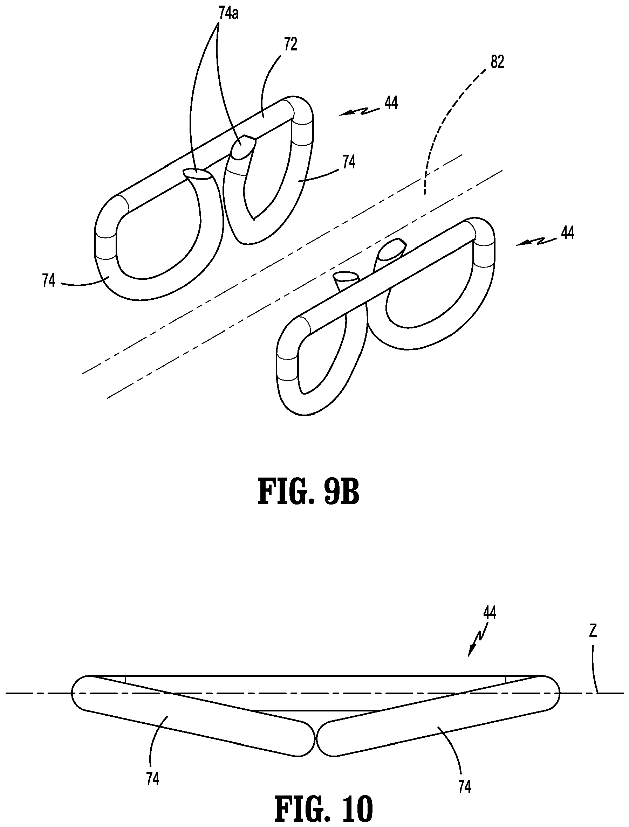

FIGS. 9A to 10 illustrate unformed and formed staples 44 of the plurality of staples 44. Each of the staples 44 includes a back span 72 and legs 74 that are supported on each end of the back span 72. Each of the legs 74 includes a tapered tip 74a. The back span 72 of the staples 44 define a longitudinal axis "Z" (FIG. 10) that is parallel to a longitudinal axis "Y" (FIG. 8) of the anvil 32 when the tool assembly 16 is in the clamped position. When the staples 44 are in an unformed condition (FIG. 9A), the legs 74 and the back span of the staples 44 are aligned within a common plane "P" (FIG. 9A).

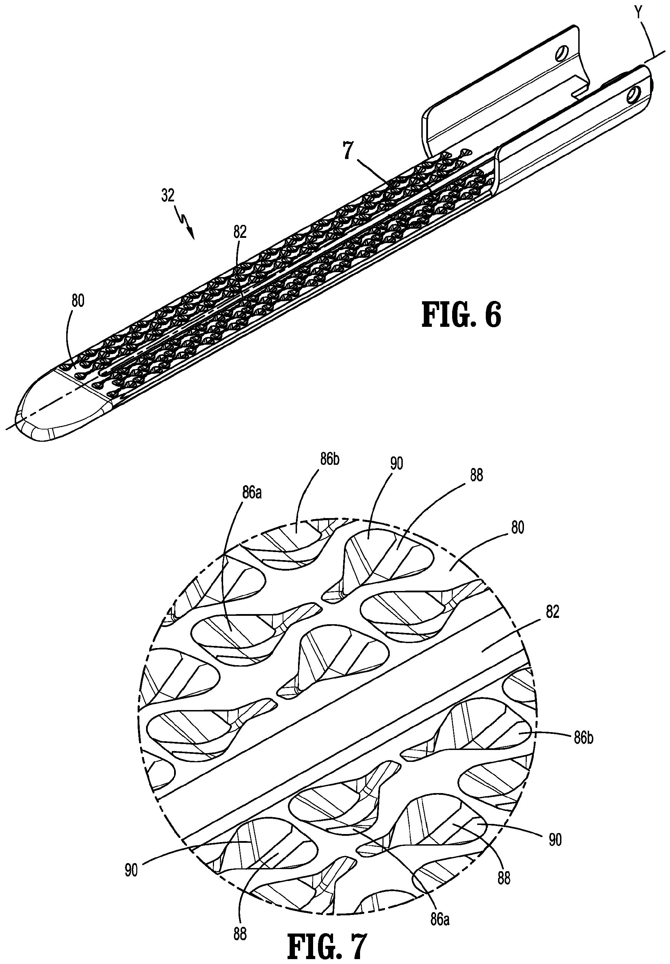

FIGS. 6-8 illustrate the anvil 32 of the tool assembly 16 which includes a body 32a having a tissue engaging surface 80 that defines a central knife slot 82 and series of staple forming pockets 84 positioned on opposite sides of the central knife slot 82. The body 32a of the anvil 32 defines a longitudinal axis "Y" (FIG. 6) and the central knife slot 82 extends along the longitudinal axis "Y". When the tool assembly 16 is in its clamped position, the tissue engaging surface 80 of the anvil 32 is in juxtaposed alignment with the tissue engaging surface 52 of the cartridge body 42 such that the staple receiving pockets 56 of the cartridge body 42 are aligned with the staple forming pockets 84 of the anvil 32.

Each of the staple forming pockets 84 includes a pair of tear-drop shape concavities 86a and 86b (FIG. 8). Each of the concavities 86a and 86b includes a linear channel 88 and tapered side walls 90 that are sloped downwardly into the linear channel 88 of the respective concavity 86a, 86b. The tapered walls 90 are positioned to receive the legs 74 of the staples 44 and direct the legs 74 of the staples 44 into the linear channel 88 of each of the concavities 86a, 86b. The linear channels 88 of the concavities 86a, 86b of each of the staple forming pockets 84 defines a longitudinal axis S1 and S2, respectively. The longitudinal axis S1 of the linear channel 88 of the concavity 86a defines an acute angle Ω1 with the longitudinal axis "Y" of the anvil 32 and the longitudinal axis S2 of the linear channel 88 of the concavity 86b defines an acute angle Ω2 with the longitudinal axis "Y" of the anvil 32. The angles Ω1 and Ω2 and are selected to direct the respective legs 74 of the staples 44 inwardly of the back span 72 such that the legs 74 when formed are positioned offset from back span 72 between the respective back span 72 and the central knife slot 82. The angles Ω1 and Ω2 inhibit contact between the legs 74 of the staples 44 and the back span 72 during staple formation which may result in staple malformation. In aspects of the disclosure, the angles Ω1 and Ω2 are from about 5 degrees to about 30 degrees. In some aspects of the disclosure, the angles Ω1 and Ω2 are substantially equal in degrees.

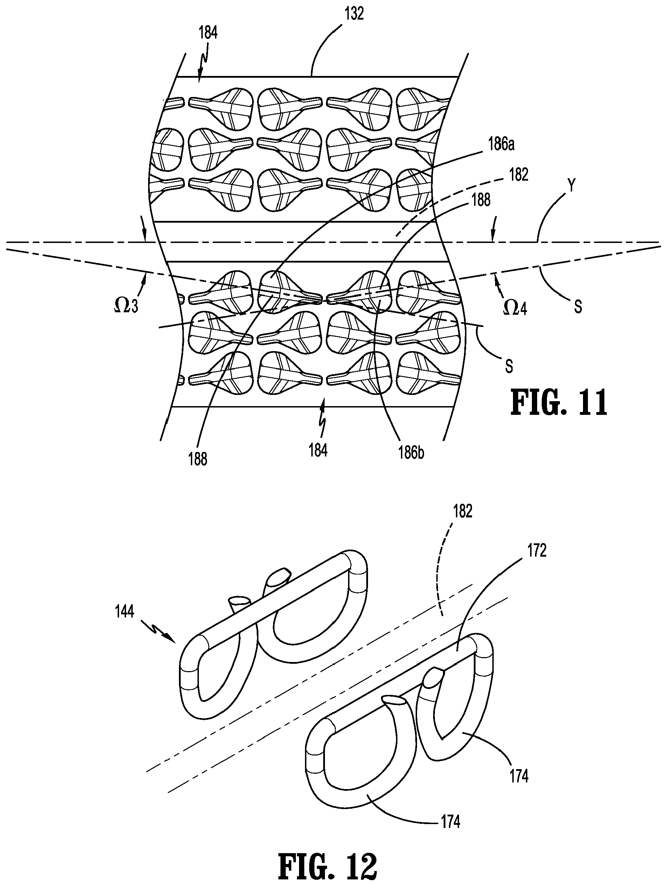

FIG. 11 illustrates an alternative version of the anvil of the stapling device of FIG. 1 shown generally as anvil 132. The anvil 132 is substantially like the anvil 32 (FIG. 7) except that the staple forming pockets 184 are configured to form the staples legs 174 (FIG. 12) to the side of the back span 172 spaced from the central knife slot 182. More specifically, the angle of the linear portion 188 of the respective tear-drop shaped concavities 186a and 186b of each of the staple pockets 184 are such that the longitudinal axis S3 of the linear channel 188 of the concavity 186a defines an acute angle Ω3 with the longitudinal axis "Y" of the anvil 132 and the longitudinal axis S4 of the linear channel 188 of the concavity 186b defines an acute angle Ω4 with the longitudinal axis "Y" of the anvil 132. In aspects of the disclosure angles Ω3 and Ω4 are from about 5 degrees to about 30 degrees. In aspects of the disclosure, angles Ω3 and Ω4 are substantially equal in degrees. The angles Ω3 and Ω4 are selected to direct the respective legs 174 of the staples 144 outwardly of the back span 172 such that the legs 174 when formed are positioned to the side of the back span 172 spaced from the central knife slot 182. The pockets controlled by the angles Ω3 and Ω4 can be designed in such a way that the staple tips 174a can be formed to on one side of the back span 172 or on the other side of the back span 172 (FIG. 12).

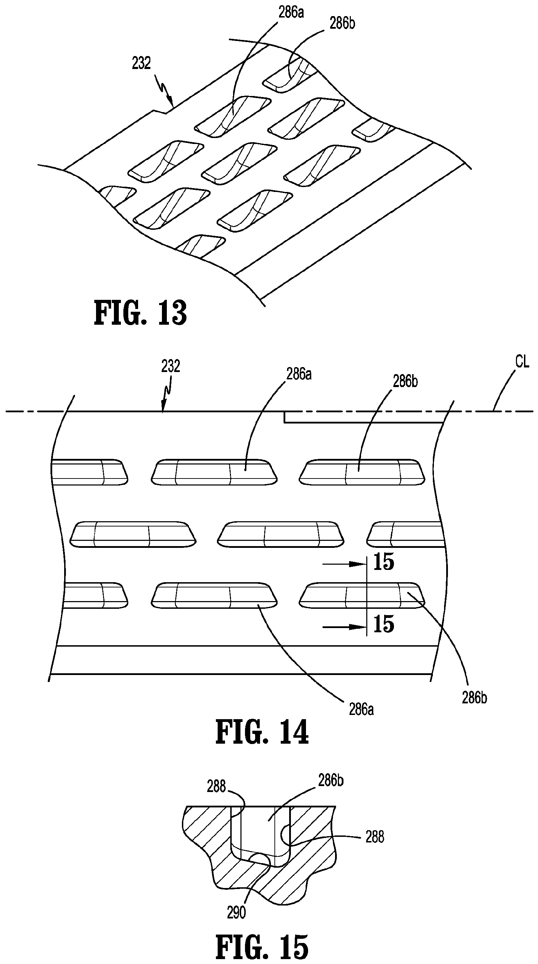

FIGS. 13-15 illustrate another version of the anvil of the stapling device of FIG. 1 shown generally as anvil 232. The anvil 232 includes anvil pockets 284 that have first and second concavities 286a and 286b. Each of the concavities 286a and 286b is substantially linear and defines a longitudinal axis that is substantially parallel to the longitudinal axis of the anvil 232. The first and second concavities 286a and 286b are defined by side walls 288 and a bottom wall 290 (FIG. 15). The bottom wall 290 is angled or skewed downwardly towards or away from the centerline CL (FIG. 14) of the anvil 232. When the legs 74 of a staple, e.g., staple 44 (FIG. 9), engage the bottom wall 290 defining a respective one of the first and second concavities 286a and 286b, the legs 74 of the staples 44 will slide downwardly along the bottom wall 290 and form outwardly of the back span 72.The bottom wall 290 defining each of the first and second concavities 286a and 286b is curved upwardly along the longitudinal axis of each of the concavities 286a and 286b to form the legs into a B-shape. As described above in regard to other aspects of the disclosure, the bottom wall of the concavities 286a and 286b can be designed in such a way that the staple tips 174a (FIG. 12) can be formed to one side of the back span 172 or to the other side of the back span 172 (FIG. 12).

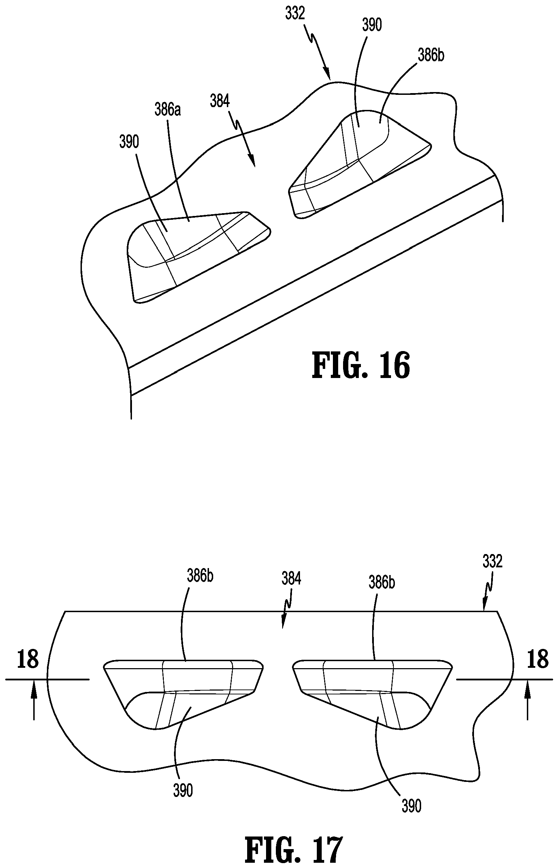

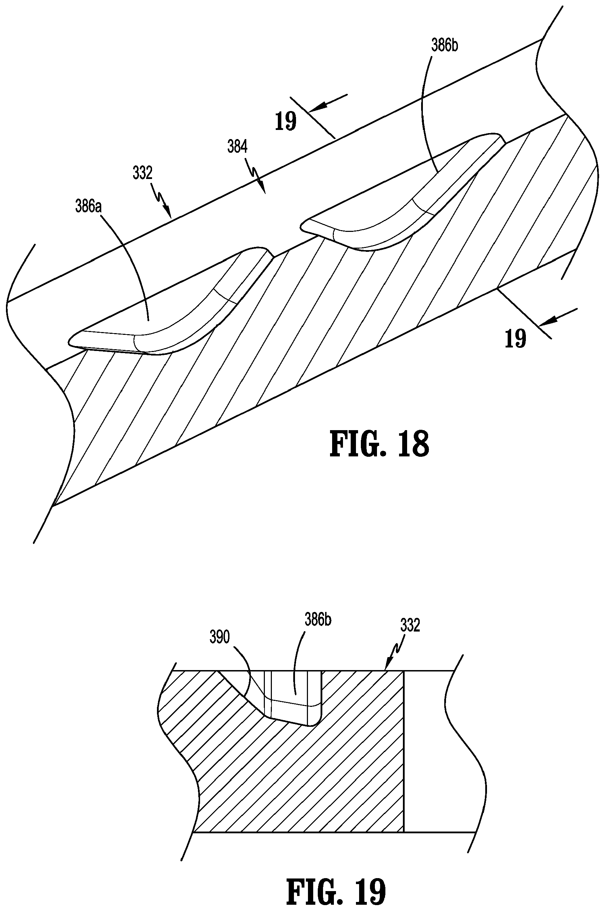

FIGS. 16-19 illustrate yet another version of the anvil of the stapling device 10 (FIG. 1) shown generally as anvil 332. The anvil 332 is substantially like the anvil 232 (FIG. 14) except that each of the first and second concavities 386a and 386b of the staple forming pockets 384 has a tear-drop shape. Each of the first and second concavities 386a and 386b includes a wall 390 that is angled or skewed downwardly towards or away from a centerline of the anvil 332. When the legs 74 of a staple, e.g., staple 44 (FIG. 9), engage the bottom wall 290 defining a respective one of the first and second concavities 386a and 386b, the legs 74 of the staples 44 will slide downwardly along the wall 390 and form outwardly of the back span 72. The wall 290 defining each of the first and second concavities 286a and 286b is curved upwardly along the longitudinal axis of each of the concavities 386a and 386b to form the legs into a B-shape.

It is noted that the staples can have the same unformed size in each of the rows of staples. Alternately, the staples can have different unformed sizes in each of the rows of staples. For example, the staples on the inner row of staples can be smaller than the staples in the outer rows of staples. In addition, the face of the tissue engaging surface 52 (FIG. 5A) of the cartridge body 42 of the staple cartridge can be planar or, alternately, it can be stepped.

Persons skilled in the art will understand that the devices and methods specifically described herein and illustrated in the accompanying drawings are non-limiting exemplary aspects of the disclosure. It is envisioned that the elements and features illustrated or described in connection with one exemplary aspects of the disclosure may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. An anvil for a surgical stapling device, the anvil comprising:
   a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive first and second legs, respectively, of a staple including a back span and configured to form the legs into a B-shape with the legs laterally offset from the back span.
2. The anvil of paragraph 1, wherein each of the first and second concavities includes tapered side walls and a linear channel, the tapered side walls configured to direct the legs of the staple into the linear channel.
3. The anvil of paragraph 2, wherein the linear channel of each of the first concavities defines an angle Ω1 with the longitudinal axis of the body of the anvil, and the linear channel of each of the second concavities defines an angle Ω2 with the longitudinal axis of the body of the anvil.
4. The anvil of paragraph 3, wherein the angles Ω1 and Ω2 are from about 5 degrees to about 30 degrees.
5. The anvil of paragraph 3, wherein each of the first and second concavities is tear-drop shaped.
6. The anvil of paragraph 1, wherein each of the first and second concavities has a wall that is angled downwardly in a direction orthogonal to the longitudinal axis of the body of the anvil.
7. The anvil of paragraph 6, wherein each of the first and second concavities is linear.
8. The anvil of paragraph 6, wherein each of the first and second concavities is tear-drop shaped.
9. A tool assembly comprising:
   a cartridge assembly including a staple cartridge and a channel member, the channel member having a bottom wall and side walls defining a cavity, the staple cartridge including a cartridge body defining a longitudinal axis, pushers, staples, and an actuation sled, the cartridge body defining a central knife slot and a series of staple receiving pockets on each side of the central knife slot, each of the staples including a back span and first and second legs secured to the back span, each of the staples received within one of the series of staple receiving slots, wherein the first and second legs of each of the staples and the back span of the respective staple are aligned in a common plane; and
   an anvil including a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive the first and second legs, respectively, of a respective one of the staples, wherein the first and second concavities are configured to form the legs into a B-shape with the first and second legs of the staples laterally offset from the back span.
10. The tool assembly of paragraph 9, wherein each of the first and second concavities includes tapered side walls and a linear channel, the tapered side walls configured to direct the legs of the staple into the linear channel.
11. The tool assembly of paragraph 10, wherein the linear channel of each of the first concavities defines an angle Ω1 with the longitudinal axis of the body of the anvil, and the linear channel of each of the second concavities defines an angle Ω2 with the longitudinal axis of the body of the anvil.
12. The tool assembly of paragraph 11, wherein the angles Ω1 and Ω2 are from about 5 degrees to about 30 degrees.
13. The tool assembly of paragraph 11, wherein each of the first and second concavities is tear-drop shaped.
14. The tool assembly of paragraph 9, wherein each of the first and second concavities has a wall that is angled downwardly in a direction orthogonal to the longitudinal axis of the body of the anvil.
15. The tool assembly of paragraph 14, wherein each of the first and second concavities is linear.
16. The tool assembly of paragraph 14, wherein each of the first and second concavities is tear-drop shaped.
17. The tool assembly of paragraph 9, wherein the cartridge assembly includes a biasing member positioned on the bottom wall of the channel member between the cartridge body and the channel member, the biasing member deformable to allow for vertical movement of the cartridge body within the cavity.
18. The tool assembly of paragraph 17, wherein the cartridge body includes tabs and the channel member defines windows that receive the tabs, the tabs being movable vertically within the windows to allow the cartridge body to move vertically in relation to the channel member.
19. The tool assembly of paragraph 17, wherein the biasing member is selected from the group consisting of compression springs and leaf springs.
20. A surgical stapling device comprising:
   a handle assembly;
   an adapter assembly having a proximal portion and a distal portion, the proximal portion coupled to the handle assembly; and
   a tool assembly including:
      a cartridge assembly including a staple cartridge and a channel member, the channel member having a bottom wall and side walls defining a cavity, the staple cartridge including a cartridge body defining a longitudinal axis, pushers, staples, and an actuation sled, the cartridge body defining a central knife slot and a series of staple receiving pockets on each side of the central knife slot, each of the staples including a back span and first and second legs secured to the back span, each of the staples received within one of the series of staple receiving slots, wherein the first and second legs of each of the staples and the back span of the respective staple are aligned in a common plane; and
      an anvil including a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive the first and second legs, respectively, of a respective one of the staples, wherein the first and second concavities are configured to form the legs into a B-shape with the first and second legs of the staples laterally offset from the back span.

## Claims

1. An anvil for a surgical stapling device, the anvil comprising:
a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive first and second legs, respectively, of a staple including a back span and configured to form the legs into a B-shape with the legs laterally offset from the back span.

2. The anvil of claim 1, wherein each of the first and second concavities includes tapered side walls and a linear channel, the tapered side walls configured to direct the legs of the staple into the linear channel.

3. The anvil of claim 2, wherein the linear channel of each of the first concavities defines an angle Ω1 with the longitudinal axis of the body of the anvil, and the linear channel of each of the second concavities defines an angle Ω2 with the longitudinal axis of the body of the anvil.

4. The anvil of claim 3, wherein the angles Ω1 and Ω2 are from about 5 degrees to about 30 degree and/or wherein each of the first and second concavities is tear-drop shaped.

5. The anvil of any preceding claim, wherein each of the first and second concavities has a wall that is angled downwardly in a direction orthogonal to the longitudinal axis of the body of the anvil.

6. The anvil of claim 5, wherein each of the first and second concavities is linear, or wherein each of the first and second concavities is tear-drop shaped.

7. A tool assembly comprising:
a cartridge assembly including a staple cartridge and a channel member, the channel member having a bottom wall and side walls defining a cavity, the staple cartridge including a cartridge body defining a longitudinal axis, pushers, staples, and an actuation sled, the cartridge body defining a central knife slot and a series of staple receiving pockets on each side of the central knife slot, each of the staples including a back span and first and second legs secured to the back span, each of the staples received within one of the series of staple receiving slots, wherein the first and second legs of each of the staples and the back span of the respective staple are aligned in a common plane; and
an anvil including a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive the first and second legs, respectively, of a respective one of the staples, wherein the first and second concavities are configured to form the legs into a B-shape with the first and second legs of the staples laterally offset from the back span.

8. The tool assembly of claim 7, wherein each of the first and second concavities includes tapered side walls and a linear channel, the tapered side walls configured to direct the legs of the staple into the linear channel.

9. The tool assembly of claim 8, wherein the linear channel of each of the first concavities defines an angle Ω1 with the longitudinal axis of the body of the anvil, and the linear channel of each of the second concavities defines an angle Ω2 with the longitudinal axis of the body of the anvil; preferably wherein the angles Ω1 and Ω2 are from about 5 degrees to about 30 degrees; preferably still wherein each of the first and second concavities is tear-drop shaped.

10. The tool assembly of claim 7, wherein each of the first and second concavities has a wall that is angled downwardly in a direction orthogonal to the longitudinal axis of the body of the anvil.

11. The tool assembly of claim 10, wherein each of the first and second concavities is linear; or wherein each of the first and second concavities is tear-drop shaped.

12. The tool assembly of any of claims 7 to 11, wherein the cartridge assembly includes a biasing member positioned on the bottom wall of the channel member between the cartridge body and the channel member, the biasing member deformable to allow for vertical movement of the cartridge body within the cavity.

13. The tool assembly of claim 12, wherein the cartridge body includes tabs and the channel member defines windows that receive the tabs, the tabs being movable vertically within the windows to allow the cartridge body to move vertically in relation to the channel member.

14. The tool assembly of claim 12 or claim 13, wherein the biasing member is selected from the group consisting of compression springs and leaf springs.

15. A surgical stapling device comprising:
a handle assembly;
an adapter assembly having a proximal portion and a distal portion, the proximal portion coupled to the handle assembly; and
a tool assembly including:
a cartridge assembly including a staple cartridge and a channel member, the channel member having a bottom wall and side walls defining a cavity, the staple cartridge including a cartridge body defining a longitudinal axis, pushers, staples, and an actuation sled, the cartridge body defining a central knife slot and a series of staple receiving pockets on each side of the central knife slot, each of the staples including a back span and first and second legs secured to the back span, each of the staples received within one of the series of staple receiving slots, wherein the first and second legs of each of the staples and the back span of the respective staple are aligned in a common plane; and
an anvil including a body defining a longitudinal axis and having a tissue engaging surface, the tissue engaging surface defining a central knife slot and a series of staple forming pockets positioned on each side of the central knife slot, each of the staple forming pockets including first and second concavities, the first and second concavities positioned to receive the first and second legs, respectively, of a respective one of the staples, wherein the first and second concavities are configured to form the legs into a B-shape with the first and second legs of the staples laterally offset from the back span.
